# EUROPEAN PATENT SPECIFICATION

(11) **EP 1 664 087 B1**
(45) Date of publication and mention of the grant of the patent: **22.08.2007**
(21) Application number: 04783316.5
(22) Date of filing: 07.09.2004
(51) Int. Cl.: C07K 1/22, G01N 33/15

(54) **PRION CLEARANCE USING PARTICULATE METAL OXIDES**
ENTFERNUNG VON PRIONEN DURCH DEN GEBRAUCH VON METALLOXID PARTIKELN
ELIMINATION DES PRIONS AU MOYEN D'OXYDES METALLIQUES PARTICULAIRES

(30) Priority: 10.09.2003 US 659789
(43) Date of publication of application: 07.06.2006
(73) Proprietor: Bayer HealthCare, LLC, Tarrytown, NY 10591 (US)
(72) Inventor: STENLAND, Christopher, J., Cary, North Carolina 27513-1651 (US); TERRY, Jarrett, C., Raleigh, North Carolina 27617 (US); YUZIUK, Jeffrey, A., Garner, North Carolina 27529 (US)
(74) Representative: Burkert, Frank
(86) International application number: PCT/US2004/029024
(87) International publication number: WO 2005/026197

(56) References cited:
- WO-A-01/00235
- WO-A-97/45746
- WO-A-02/090379
- US-A- 5 808 011
- US-A1- 2004 171 103
- PAN K-M ET AL: "PURIFICATION AND PROPERTIES OF THE CELLULAR PRION PROTEIN FROM SYRIAN HAMSTER BRAIN" PROTEIN SCIENCE, CAMBRIDGE UNIVERSITY PRESS, CAMBRIDGE, GB, vol. 1, 1992, pages 1343-1352, XP002043228 ISSN: 0961-8368
- VAN HOLTEN ET AL.,: "Removal of Prion Challenge From an Immune Globulin Preparation by Use of a Size-exclusion Filter" TRANSFUSION, vol. 42, 1 August 2002 (2002-08-01), pages 999-1004, XP002305022
- LEE D C ET AL: "MONITORING PLASMA PROCESSING STEPS WITH A SENSITIVE WESTERN BLOT ASSAY FOR THE DETECTION OF THE PRION PROTEIN" JOURNAL OF VIROLOGICAL METHODS, AMSTERDAM, NL, vol. 84, no. 1, January 2000 (2000-01), pages 77-89, XP001176838 ISSN: 0166-0934

## Description

### FIELD OF THE INVENTION

The invention relates to the separation or clearance of pathogenic prion proteins from biological materials. The pathogenic prions are separated from biological materials by exposing the material to metal oxides and/or particulate silicon dioxide, including fumed metal oxides such as fumed silica.

### BACKGROUND

Pathogenic prion protein (pathogenic prions) are associated with transmissible spongiform encephalopathies (TSEs), which are fatal neurodegenerative diseases in humans and other mammalian species, particularly sheep, goats, cattle, mink, elk, and deer. Human forms of the disease include Creutzfeldt-Jakob disease (CJD), Gerstmann-Straussler-Scheinker (GSS) syndrome, fatal familial insomnia (FFI), and Kuru. Symptoms of these diseases include myoclonus, ataxia, and progressive dementia. The pathology of these diseases includes the formation of amyloid plaques in the brain.

The occurrence of prion diseases has been linked to infectious transmission, as well as genetic and sporadic causes. For example, Kuru, which afflicted the Fore tribe of New Guinea, was caused by the ingestion of infected brain tissue of other tribal members during ritualistic cannibalism. Alpers, "Slow Transmissible Diseases of the Nervous System," Vol. 1, S. B. Pruisner and W. J. Hadlow, eds. (New York: Academic Press), pp. 66-90 (1979). More recently, the occurrence of variant CJD (vCJD) in the United Kingdom has been postulated to be the result of consumption of beef from cattle infected with a pathogenic prion. G. Chazot, et al., Lancet 347:1181 (1996); R. G. Will, et al., Lancet 347:921-25 (1996). Similarly, iatrogenic CJD has been caused by the injection of human growth hormone derived from cadaveric pituitaries. Brown, et al., Lancet 340:24-27 (1992). Instances of infectious activity of prions, as noted above, suggest that prion infectivity may be transmitted through biological materials.

Prions are believed to infect humans and other mammals via transmission of a protein, the prion protein, without concomitant transmission of a nucleic acid. It has been postulated that the infectious agent is a disease-causing variant of the naturally-occurring, cellular prion protein (PrP^{C}), the physiologic function of which is presently not known. In this theory, the variant form of the prion protein (PrP^{Sc}) induces structural changes in the normal form of the prion protein (PrP^{C}), thereby converting it to the disease-causing form of the pathogenic prion protein (PrP^{Sc}). PrP^{Sc} may be detected by subjecting a sample to proteinase K treatment and analyzing the sample for the presence of the proteinase resistant, polypeptide (PrP^{RES}). See U.S. Patent No. 6,437,102 to Lee, et al.*,* assigned to Bayer Corporation; and Lee, D.C., et al., "Monitoring plasma processing steps with a sensitive Western blot assay for the detection of the prion protein," J Virol Methods, 84(1):77-89(2000).

Therapeutic compositions are often derived from biological materials. For example, therapeutically valuable proteins such as Factor VIII, immunoglobulins, α-1 proteinase inhibitor, plasminogen/plasmin, and albumin are isolated from human plasma. Other infectious agents, such as human immunodeficiency virus (HIV) and hepatitis C virus (HCV) have been known to be transmitted by therapeutic use of blood or blood products that have not undergone inactivation methods or procedures that effectively remove the agents from the product processing stream. Evidence of the transmission of TSEs through other biological materials raises concern over possible transmission of TSEs through blood and blood-derived products, recombinant products, and other products of biological origin.

Known methods for reducing the risk of infection from blood borne pathogens, such as HIV and HCV, however, are not effective at inactivating prions. Furthermore, methods that do reduce the risk of infection from prions, which include treatment with strong base and/or autoclaving, are not suited for use with procedures for the preparation of therapeutic or otherwise useful proteins, because such procedures denature the proteins.

A method for separating prions from biological materials, such as blood plasma fractions and aqueous solutions of recombinantly produced proteins, is therefore needed. The present invention provides a method of separating prions from such biological materials without denaturing therapeutically valuable proteins.

### SUMMARY OF THE INVENTION

The invention provides methods for the separation or clearance of prions from biological materials. It will be recognized that such methods are useful for ensuring that products originating from biological materials are safe for subsequent use by humans or animals that might become infected with pathogenic prion protein derived from the original material.

Accordingly, in one aspect the invention relates to a method of preparing a solution containing biological material by adding a metal oxide to biological material to obtain a solution comprising a mixture of the metal oxide and the biological material; and separating the metal oxide from the mixture to form a resulting solution. Pathogenic prion proteins possibly contaminating the biological material are substantially reduced in the resulting solution. In some embodiments, the method can include evaluating the resulting solution for the presence or amount of pathogenic prion protein.

In another embodiment, the invention relates to a method of preparing a solution containing biological material by adding fumed silica characterized by a specific surface area of from about 150 m2/g to about 300 m2/g to biological material to obtain a solution comprising a mixture of fumed silica and the biological material; separating the fumed silica from the mixture to form a resulting solution by passing the mixture through a filtration system comprising a filter which retains at least a substantial portion of particles of the fumed silica; and evaluating the resulting solution for the presence or amount of pathogenic prion protein using an immunoassay. Pathogenic prion proteins possibly contaminating the biological material are substantially reduced in the resulting solution.

In another aspect, the invention relates to a method of preparing a solution containing biological material by adding silicon dioxide particles to biological material to obtain a solution comprising a mixture of silicon dioxide particles and the biological material; separating the silicon dioxide particles from the mixture to form a resulting solution; and evaluating the resulting solution for the presence or amount of pathogenic prion protein. Pathogenic prion proteins possibly contaminating the biological material are substantially reduced in the resulting solution.

The invention also relates to a method of separating prions from a sample by contacting a sample in a flowable liquid state with a solid substrate comprising a metal oxide; allowing the sample to remain in contact with the substrate for a time such that prions in the sample bind to the substrate; and separating the sample from the substrate.

The invention further relates to a method of separating prion proteins from a sample and concentrating them for further analysis by contacting the sample with a particulate metal oxide; separating the particulate metal oxide from the sample; and subjecting prion proteins associated with the particulate metal oxide to further analysis.

### BRIEF DESCRIPTION OF THE FIGURES

Figure 1 shows Western blots illustrating specific detection of pathogenic prion protein (PrP^{Sc}) following filtration using CAB-O-SIL (fumed silica) at the concentrations indicated adjacent to the panels. Materials and methods utilized are as indicated in Example 1.
Figure 2 is a graphic representation of the clearance or removal of PrP^{Sc} using various amounts of added CAB-O-SIL. The data used to generate the graph is represented in the Western blot experiments as shown in Figure 1.
Figure 3 shows Western blots illustrating PrP^{Sc} clearance accomplished by addition of CAB-O-SIL during purification of plasminogen. See Example 2 for details regarding materials and methods.
Figure 4 shows Western blots illustrating PrP^{Sc} clearance accomplished by addition of CAB-O-SIL during a variation of the process for purification of plasminogen involved in generating the results shown in Figure 3. See Example 2 for details regarding materials and methods.
Figure 5 shows Western blots illustrating PrP^{Sc} clearance accomplished by addition of Al(OH)₃. Lanes represent analysis of samples having Al(OH)₃ added (% v/v of ANHYDROGEL) as follows: a, 0; b, 1; c, 2; d, 5; e, 10; and f, 18. See Example 3 for details regarding materials and methods.

### DETAILED DESCRIPTION

The present invention provides a method for preparing solutions wherein pathogenic prion proteins potentially contaminating the solutions are substantially reduced relative to a starting solution. The invention also provides for the preparation of prion proteins for further analysis based on their recovery from contaminated solutions.

Accordingly, in one aspect, the invention relates to a method of preparing a solution containing biological material by adding a metal oxide to biological material to obtain a solution comprising a mixture of the metal oxide and the biological material; and separating the metal oxide from the mixture to form a resulting solution. In some embodiments, the method further comprises evaluating the resulting solution for the presence or amount of pathogenic prion protein.

In particular embodiments, the biological material can be blood-derived products, tissue-derived products, or recombinantly produced products. In some embodiments, the biological material is a blood-derived product. The blood-derived product can be of human origin. The blood-derived product can be immunoglobulins, blood coagulation factors, plasmin, plasminogen, α-1 proteinase inhibitor, or albumin.

In particular embodiments, the metal oxide can be fumed silica or fumed alumina. The fumed metal oxide can be fumed silica. The fumed silica can be characterized by a specific surface area of from about 130 m²/g to about 380 m²/g. In particular embodiments, the fumed silica can be characterized by a specific surface area of from about 150 m²/g to about 300 m²/g. The fumed silica can also be characterized by a specific surface area of about 200 m²/g.

In some embodiments, the metal oxide is separated from the mixture by filtration.
The filtration can include passing the mixture through a filtration system that retains particles larger than from about 0.1 mm to about 5 mm. The filtration can also include passing the mixture through a filtration system that retains particles larger than about 0.8 µm.

Evaluating the resulting solution for the presence or amount of pathogenic prion protein can include evaluating a sample for infectivity using an animal bioassay or an immunoassay for the pathogenic prion protein. The immunoassay can be a Western blot or ELISA assay.

In some embodiments, the metal oxide can be aluminum hydroxide. The aluminum hydroxide, as a gel comprising about 2% by weight Al₂O₃, can be present at a concentration from about 1% to about 10% by volume, from about 1% to about 5% by volume, or at about 3% by volume.

In other embodiments, the metal oxide can be fumed alumina. The fumed alumina can be present in an amount of from about 0.1% to about 1.0% by weight. The fumed alumina can also be present in an amount from about 0.25% to about 0.75% by weight. The fumed alumina can also be present in an amount of about 0.5% by weight.

In yet another aspect, the invention relates to a method of preparing a solution containing biological material by adding fumed silica characterized by a specific surface area of from about 150 m²/g to about 300 m²/g to biological material to obtain a solution comprising a mixture of fumed silica and the biological material; separating the fumed silica from the mixture to form a resulting solution by passing the mixture through a filtration system comprising a filter which retains at least a substantial portion of particles of the fumed silica; and evaluating the resulting solution for the presence or amount of pathogenic prion protein using an immunoassay.

In some embodiments, the fumed silica is characterized by a specific surface area of about 200 m²/g, a tap density of about 50 g/l, and an average aggregate particle length of from about 0.2 µm to about 0.3 µm. The fumed silica is added in an amount from about 0.1% to about 1.0% (weight/weight) of the solution comprising a mixture of fumed silica and the biological material. The fumed silica can be added in an amount from about 0.2% to about 0.8% (weight/weight) of the solution comprising a mixture of fumed silica and the biological material. The fumed silica can also be added in an amount of at least about 0.25% (weight/weight) of the solution comprising a mixture of fumed silica and the biological material. In particular embodiments, the fumed silica can be added in an amount of at least about 0.5% (weight/weight) of the solution comprising a mixture of fumed silica and the biological material.

In another aspect, the invention relates to a method of preparing a solution containing biological material by adding silicon dioxide particles to biological material to obtain a solution comprising a mixture of silicon dioxide particles and the biological material; separating the silicon dioxide particles from the mixture to form a resulting solution; and evaluating the resulting solution for the presence or amount of pathogenic prion protein. Pathogenic prion proteins possibly contaminating the biological material are substantially reduced in the resulting solution.

In some embodiments, the separating of silicon dioxide particles from the mixture is accomplished by centrifugation or filtration.

In another aspect, the invention relates to a method of separating prions from a sample by contacting a sample in a flowable liquid state with a solid substrate comprising a metal oxide; allowing the sample to remain in contact with the substrate for a time such that prions in the sample bind to the substrate; and separating the sample from the substrate. The metal oxide can be silicon dioxide or aluminum hydroxide. The metal oxide can by fumed silica.

In another aspect, the invention relates to a method of separating prion proteins from a sample and concentrating them for further analysis, the method by contacting the sample with a particulate metal oxide; separating the particulate metal oxide from the sample; subjecting prion proteins associated with the particulate metal oxide to further analysis. The analysis of prion proteins can include immunoassay, animal bioassay, spectroscopic analysis, or chromatographic analysis.

The invention provides methods for the separation or clearance of prions from biological materials. Biological materials include biologically derived fluids, such as blood plasma fractions, biological samples, such as samples of tissues, and aqueous solutions of recombinantly produced products, such as recombinant proteins. Generally, the methods provide for the separation of prions from the biological material by adding a particulate silicon dioxide or metal oxide to the biological material to obtain a mixture of the particulate silicon dioxide or metal oxide and the biological material. The particulate silicon dioxide or metal oxide is then separated from the mixture, such as by filtration or centrifugation, to obtain a purified biological material that is characterized by a substantial reduction in any pathogenic prion proteins that may have contaminated the original biological material.

It will be recognized that such methods are useful for ensuring that products originating from biological materials are safe for subsequent use by humans or animals that might become infected with pathogenic prion protein derived from the original material. Accordingly, the practice of the invention includes carrying out the disclosed method on biological materials that may or may not actually comprise pathogenic prion protein as a quality assurance method for the processing of potentially contaminated biological materials.

As used herein, the following terms have the indicated meaning unless otherwise expressly indicated:

"Animal bioassay" means an assay for pathogenic prion in a material involving the introduction of a test sample of the material into a living organism susceptible to disease caused by or associated with pathogenic prion proteins, followed by monitoring of the organism for evidence of the development of a related disease state. Animal bioassays include, but are not limited to, the rodent infectivity assay as described by Brown, P., et al., "The distribution of infectivity in blood components and plasma derivatives in experimental models of transmissible spongiform encephalopathy," Transfusion, 38(9):810-6 (1998).

"Blood-derived product" means any product produced from whole or fractionated blood sources, generally mammalian.

"Biological material" means any material originating from a living organism that can be processed as a solution. Biological material that can be subjected to the method of the invention can include any biological material but will generally be a material that, because of its origin, processing history, or other exposure, is potentially contaminated with pathogenic prion protein.

"Metal oxide" means a metal oxide in small particle form, having a large surface area relative to weight. Fumed metal oxides are included, usually formed by injection of a metal chloride into a flame of hydrogen and oxygen or air ("fumed" or pyrogenic metal oxides). Generally, such material is amorphous but can comprise mixtures of various crystalline phases. Particulate silicon dioxide and aluminum hydroxide are included. Such particles can form three-dimensional aggregates of primary particles. Fumed metal oxides include fumed silica and fumed alumina.

"Fumed silica" means pyrogenic, amorphous colloidal silicon dioxide.

"Immunoassay" means an assay based on specific and detectable interaction between an antibody and prion protein. If the immunoassay itself is nonspecific with respect to nonpathogenic and pathogenic isoforms of the prion protein, its use can be coupled to a procedure that substantially distinguishes between the isoforms, e.g. by proteolytic digestion of the nonpathogenic isoform. Examples of useful immunoassays include Western blots, ELISAs, and dot blot assays.

''Pathogenic prion" or "PrP^{Sc}" means any proteinaceous infectious agent associated with any of a variety of transmissible neurodegenerative diseases affecting humans or other mammals, including currently characterized forms of human transmissible spongiform encephalopathies (*e.g.,* variant Creutzfeldt-Jakob Disease (vCJD), kuru, Gerstmann-Strassler-Scheinker Disease (GSS), and fatal familial insomnia (FFI)), bovine spongiform encephalopathies ("mad cow" disease), scrapie (affecting sheep and goats), chronic wasting disease (deer), as well as other partially characterized and as yet uncharacterized diseases spread through exposure to a pathogenic form (scrapie isoform - PrP^{Sc}) of a prion protein (see Prusiner, S. B., "Molecular biology of prion disease," Science 252:1515-1522 (1991)).

"Recombinantly produced product" means any product produced using any man-made nucleic acid construct (such as constructs used to generate transgenic organisms altered to produce a protein product not normally produced, or not produced in a particular amount, in the native organism). Such products can be potentially contaminated with pathogenic prion protein due to any exposure to animals used in the recombinant production process or otherwise. It will be recognized that "recombinant" means originally produced by recombinant methods, and that subsequent production of the recombinant products can be carried out in a manner that is, in and of itself, technically nonrecombinant (e.g., a transgenic animal originally produced through recombinant technology and subsequently propagated for production of the recombinant product).

"Silicon dioxide" means fine particle size, high surface area silicon dioxide particles or aggregates of particles.

"Specific surface area" means the surface area per unit weight of a particulate solid, *e.g.* as determined by the B.E.T. (Brunauer, Emmett, and Teller) method.

"Tissue-derived product" means any product produced from tissue of a living organism, including those products produced for therapeutic or nutritional use, the processing of which involves, in at least one aspect, phase of manufacture, or handling step, the production of a solution capable of forming a mixture with a metal oxide or particulate silicon dioxide or aluminum hydroxide.

The fumed metal oxide for use in methods of the invention can be fumed silica or fumed alumina. The particulate silicon dioxide that can be used according to at least some embodiments of the method of the invention is characterized as a very fine primary particle size. Average aggregate particle size can be more difficult to determine conclusively and consistently. In some embodiments of the invention, the silicon dioxide utilized is a colloidal silicon dioxide. Colloidal silicon dioxide is a submicron fumed silica prepared by the vapor-phase hydrolysis (*e.g*., at 1110° C) of a silicon compound, such as silicon tetrachloride. Such products are commercially available from a number of sources, including Cabot Corporation, Tuscola, IL (under the tradename CAB-O-SIL) and Degussa, Inc., Piscataway, NJ (under the tradename AEROSIL).

Colloidal silicon dioxide is also known as colloidal silica, fumed silica, light anhydrous silicic acid, silicic anhydride, and silicon dioxide fumed, among others. A variety of commercial grades of colloidal silicon dioxide are produced by varying the manufacturing process. Such modifications do not typically affect the silica content, specific gravity, refractive index, color or amorphous form. However, these modifications can change the particle size, surface areas, and bulk densities of the colloidal silicon dioxide products.

The surface area of one class of silicon dioxide used in particular embodiments of the method of the invention ranges from about 50 m²/g to about 500 m²/g. The average primary particle diameter of the preferred class of silicon dioxides utilized in the invention ranges from about 5 nm to about 50 nm. However, in commercial colloidal silicon dioxide products, these particles are agglomerated or aggregated to varying extents. Average aggregate particle size (length) can be from about 100 nm (0.1 µm) to about 500 nm (0.5 µm). The tap density of the one class of silicon dioxides utilized in the invention ranges from about 20 g/l to about 100 g/l.

Commercially available colloidal silicon dioxide products can have, for example, a BET surface area ranging from about 50 m²/g to about 400 m²/g. Commercially available particle sizes can range from a nominal particle diameter of about 7 nm to an average primary particle size of about 40 nm. These commercially available products are described for exemplification purposes of acceptable properties of a useful class of silicon dioxides only, and this description is not meant to limit the scope of the invention in any manner whatsoever.

The method of the invention can be used to separate prions from blood plasma fractions. When used to separate prions from blood plasma fractions, the method of the invention can result in from at least about 2 to at least about 3 logs of clearance of prions from the blood plasma fraction.

The method of the invention can be used to separate prions from aqueous solutions of recombinantly produced products. A recombinantly produced product can be a recombinantly produced protein.

In particular embodiments of the methods of the invention, any pathogenic prion protein potentially contaminating a blood plasma fraction can be substantially reduced by adding particulate silicon dioxide to the blood plasma fraction to form a mixture. In particular embodiments, the particulate silicon dioxide is added to about 0.5 % (weight/weight) of the mixture. The particulate silicon dioxide is then removed from this mixture and the resulting solution has substantially reduced prion contamination relative to the starting material. The silicon dioxide may be removed by filtration in certain embodiments.

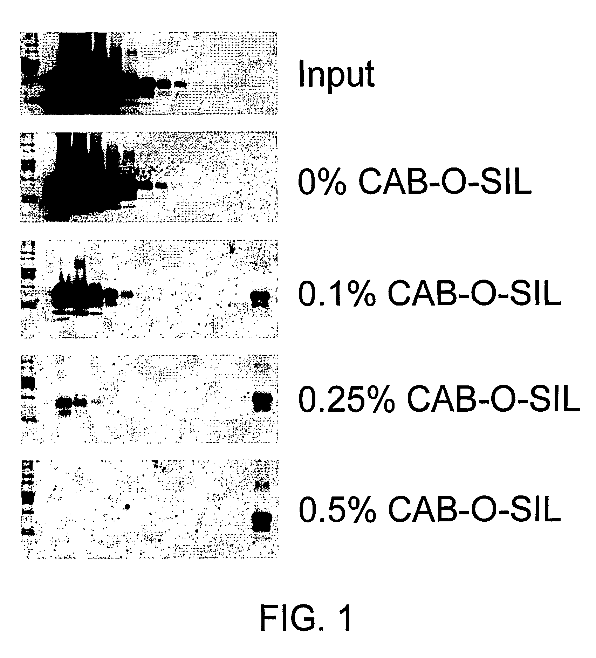

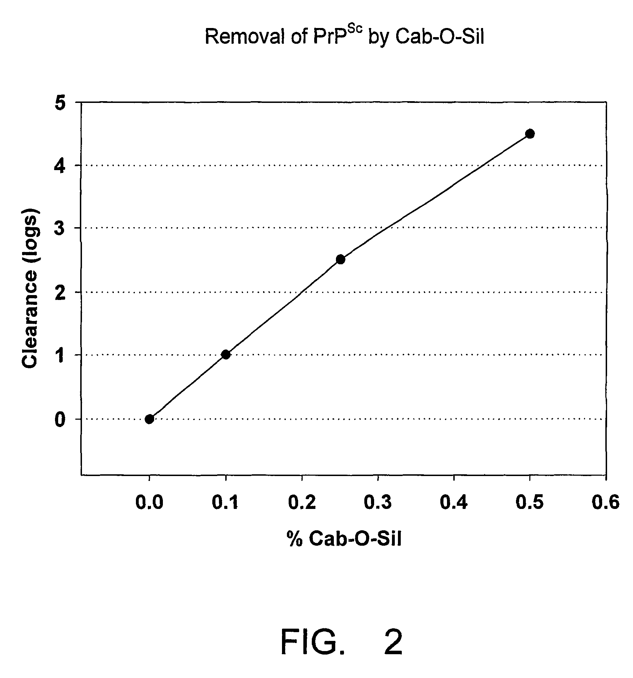

The results achieved according to one embodiment of the present invention may be evaluated by using a Western blot assay to measure the partitioning of the removal of PrP^{Sc} by the addition of CAB-O-SIL M-5P to a solution of bovine serum albumin (BSA) spiked with clarified scrapie brain homogenate (SBH). According to the present invention, clearance to the limit of detection, approximately 4.5 logs, can be achieved by adding 0.5% CAB-O-SIL followed by removal of CAB-O-SIL using 0.8 µm filtration (no more than approximately 0.5 log clearance is attributable to the 0.8 µm filtration. itself). See Example 1, as well as Figures 1 and 2.

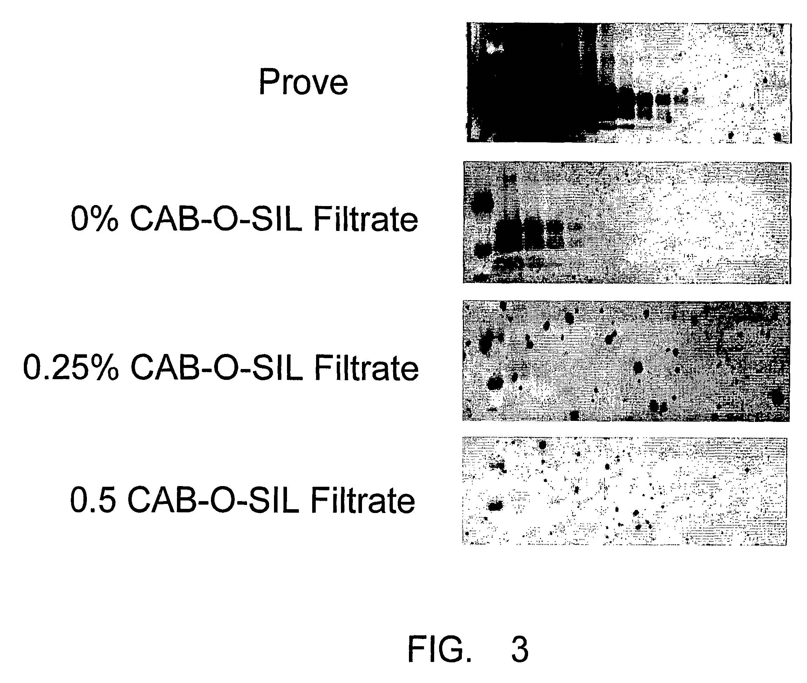

Further, the addition of CAB-O-SIL fumed silica (about 0.25% weight/weight) during a process for purification of plasminogen results in at least about 2-3 additional logs clearance of pathogenic prion protein. Example 2 describes the experiments conducted to evaluate prion clearance in this context, while Figure 3 shows Western blot analysis of the results. The data show improved clearance of 3 logs over samples processed with no fumed silica additive.

It will be recognized that a sample subjected to prion clearance according to the methods of the invention may be exposed to a metal oxide as a suspension in a liquid sample, or a liquid, flowable sample may be exposed to the metal oxide in the form of a solid substrate. The cleared sample may be recovered by a variety of means, including as a filtrate using various filtration protocols, as a supernatant using centrifugation, or as an eluate from a solid substrate.

The following Examples are illustrative of particular embodiments of the invention, but should not be viewed as limiting of the scope of the invention as claimed herein.

### EXAMPLES

### Example 1 PrP^{Sc} Clearance Using Various Concentrations of CAB-O-SIL M-5P

Bovine serum albumin (BSA) was dissolved in phosphate buffered saline (PBS) to create a solution at 1 mg/ml BSA. The BSA solution was "spiked" with scrapie brain homogenate (SBH; prepared using hamster brains infected with the 263K hamster-adapted agent), highly clarified prior to use by centrifugation at 10,000g for 10 minutes to a final concentration of approximately 1%. CAB-O-SIL M-5P silica (CAB-O-SIL) was added at various concentrations, followed by vortexing and filtration using a 0.8 µm filter (filtration alone was estimated to account for approximately 0.5 log reduction in PrP^{Sc}). CAB-O-SIL was substantially retained by the filter.

Western blot analyses of Input Sample, 0%, 0.1%, 0.25% and 0.5% CAB-O-SIL filtrates are shown in Figure 1. Figure 2 shows a graphic representation of the effect of increasing concentration of CAB-O-SIL on PrP^{Sc} clearance. (For materials and methods in detail, see U.S. Patent No. 6,437,102 to Lee, et al.*,* assigned to Bayer Corporation; and Lee, D.C., et al., "Monitoring plasma processing steps with a sensitive Western blot assay for the detection of the prion protein," J Virol Methods, 84(1):77-89(2000), providing details regarding sensitive Western blot analysis for the detection of prion proteins; see also, Stenland, C.J., et al., "Partitioning of human and sheep forms of the pathogenic prion protein during the purification of therapeutic proteins from human plasma," Transfusion, 42(11):1497-500 (Nov 2002), indicating that partitioning of human prions is similar to that observed in the hamster scrapie model; and Lee, D.C., et al., "A direct relationship between the partitioning of the pathogenic prion protein and transmissible spongiform encephalopathy infectivity during the purification of plasma proteins," Transfusion, 41(4):449-55 (2001), indicating that prions detected by the disclosed methods correlate with animal infectivity).

The experiments above, with results as shown in Figure 1, were repeated except that a highly purified, non-membrane associated scrapie brain spike ("Bolton preparation," see Bolton et al. , Arch Biochem Biophys, 258:579-90 (1987)) was used at approximately 0.5% final concentration. Western analyses of Input, 0% CAB-O-SIL, 0.1%, 0.25% and 0.5% yielded substantially identical results, with clearance to the limit of detection resulting from use of 0.5% CAB-O-SIL. However, it was noted that, in contrast to the 0.5 log clearance by 0.8 µm filtration of the clarified scrapie brain homogenate (of the total 5 logs achieved), 2.5 logs clearance of the Bolton preparation was due to filtration alone.

### Example 2 PrP^{Sc} Clearance Using CAB-O-SIL in Plasminogen Purification Process

Details of a plasmin/plasminogen purification process, proprietary to the assignee of the present application, are disclosed in a copending U.S. Patent Application, entitled "Process for Production of Reversibly Inactive Acidified Plasmin Composition" (U.S. Patent Application Publication No. US 20020192794 A1 to Dadd, *et al.*)*.* The starting material for the process step involving use of fumed silica is a paste formed during the purification of immunoglobulin from Cohn Fractions II and III, a caprylate precipitate referred to herein as Caprylate Cake I (CCI).

CCI was extracted with 200mM L-lysine and 0.1M Tris-HCl buffer for three hours. Two percent HYFLO SUPERCEL (filter aid - Celite Corporation, Lompoc, CA) was added, and the suspension was filtered through a filter cloth to remove spent filter aid. The scrapie brain homogenate spike was added after the cloth filtration and prior to the 3% polyethylene glycol 3350 (PEG) additions. The resulting spiked filtrate was divided into 3 aliquots. One portion received no CAB-O-SIL, the second received 0.25%, and the third aliquot received 0.5% (w/w) CAB-O-SIL. Following another 3 hour mix, 2% (w/w) filter aid was added, and the material was filtered through a CUNO SP-90 filter pad. The resulting filtrates were analyzed by Western blot for PrP content.

The results are shown in Figure 3. The results appear to indicate 0.5 to 1.0 log of signal present in the 0.25% and the 0.5% CAB-O-SIL filtrates. On subsequent investigation using control studies, the signals were shown to be non-specific IgG reactions with the secondary antibody, and not PrP related. Although CAB-O-SIL removed PrP from the CCI extract material, comparable protein levels including plasminogen concentrations were observed for all three CAB-O-SIL addition levels. Thus, CAB-O-SIL removed the PrP with minimal affect on the desired protein components.

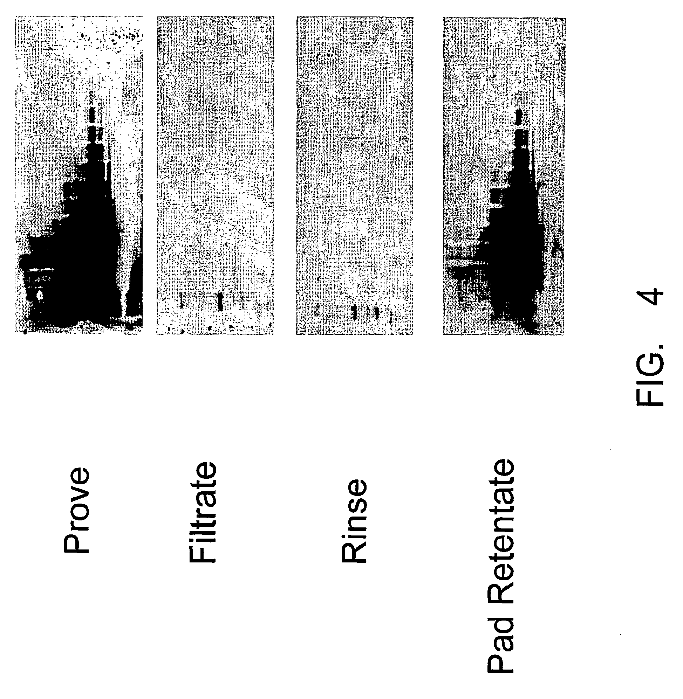

In other experiments, the CCI was extracted using 50mM dibasic sodium phosphate containing 200mM L-Lysine, 4% PEG, and 0.25% CAB-O-SIL. Ten percent crude brain homogenate was added at the beginning of the extraction and mixed over 4 hours. Filter aid was added and the solution filtered through a CUNO SP90.
A signal appears in the filtrate and rinse (Figure 4). As above, control studies verified that these signals were not PrP specific. The PrP was retained in the filter aid and on the filter pad.

### Example 3 Al(OH)₃ for Prion Clearance

As in Example 1, 0.1% BSA/TBS was spiked with SBH to final concentrations of 1% or 0.1%. These samples were used to evaluate aluminum hydroxide (Al₂O₃, 1.9-2.2% (w/v) as a gel or slurry- represented also as Al(OH)₃ or aluminum hydroxide herein) (ALHYDROGEL, Superfos Biosector A/S, Denmark) as an agent useful for prion clearance. The volume/volume percentages below refer to the proportion of the ALHYROGEL product added

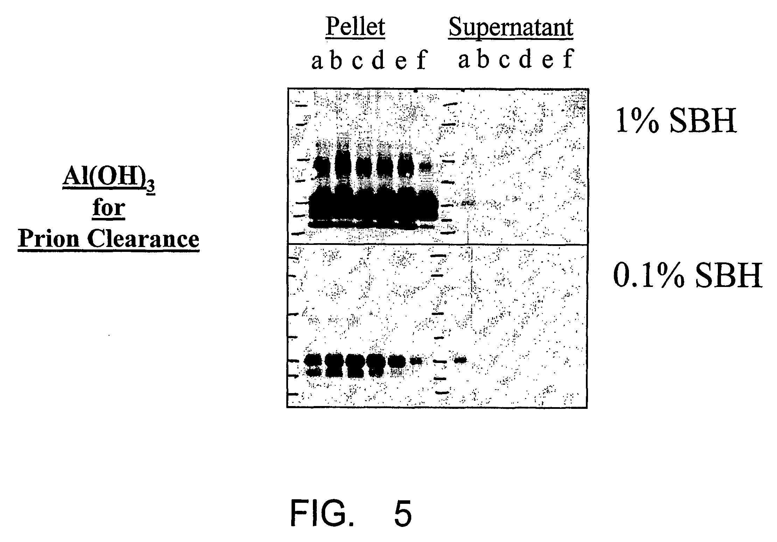

Various amounts of Al(OH)₃ (final concentrations of 0 to 18% (v/v) as indicated in Table 1) were added to samples containing SBH, and the samples were mixed as in Example 1. The samples were then centrifuged at 5100 g for 5 minutes, and the supernatant and pellet were assayed for PrP^{Sc}. In Figure 5, the lanes represent analysis of samples having Al(OH)₃ added (% v/v) as in Table 1.

**Table 1. Concentration of Al(OH)₃**

| **lane** | **% Al(OH)₃ slurry (v/v)** |
|---|---|
| a | 0.0 |
| b | 0.9 |
| c | 1.8 |
| d | 4.5 |
| e | 9.1 |
| f | 18.2 |

For the 1 % SBH, clearance was greater than 4 logs for aluminum hydroxide when treated with more than 4.5% (v/v). For 0.1% SBH clearance was greater than 3 logs for aluminum hydroxide greater than 1 % (v/v).

### Example 4 Evaluation of Plasminogen Extraction Process

In order to validate a model system for evaluating PrP^{Sc} clearance according to a particular embodiment of the present invention, a scaled-down model for Caprylate Cake I (CCI) extraction (as discussed above regarding plasminogen purification procedure) was characterized with respect to the clearance effect of the PEG Precipitation/Depth Filtration Steps. The purpose of this study was to establish a bench-scale model of the CCI Extraction and PEG Precipitation/Depth Filtration step in the Plasminogen Process under standard conditions. Once established, the model system was used to evaluate PrP^{Sc} clearance across the process step.

Briefly, CCI was resuspended in 0.1 M TRIZMA base extraction buffer (pH 10.5) at 4° C while mixing for 2-3 hours. Following extraction, the pH of the solution was adjusted to 7.5 and the temperature of the extract increased to 20° C. L-lysine was added to the extract to a final concentration of 100mM, while maintaining a pH of 7.5. Polyethylene glycol (PEG) was added to a final concentration of 3% (w/w) followed by the addition of HYFLO SUPERCEL filter aid to a final concentration of 4% (w/w). The extract was then filtered through a CUNO SP-30 filter pad and filtrate collected. Samples were collected from the initial CCI extract, filtrate, and extract. Total protein was determined by A₂₈₀ and plasminogen recovery was determined by immunonephelometry. Recovery analysis indicated very little protein loss across this step.

Next, PrP^{Sc} clearance during the CCI and PEG precipitation/depth filtration step was evaluated. The purpose of this experiment was to determine the amount of PrP^{Sc} removed during the extraction of the CCI and PEG precipitation/depth filtration steps.

The protocol was the same as described above, except that during the extraction of CCI, 1 ml of 10% crude SBH was added into 100 ml of the extract resulting in 0.1 % final SBH concentration. The paste retained by the CUNO SP-30 filter was resuspended to original volume in TBS. Samples from the Prove (spiked extract prior to filtration), filtrate and paste resuspension were analyzed for both plasminogen and PrP^{Sc} by Western analysis.

The steps above (no aluminum hydroxide or CAB-O-SIL) resulted in 1 log of clearance PrP^{Sc}.

### Example 5 Effect of Al(OH)₃ on Plasminogen Recovery and PrP^{Sc} Clearance

The effect of 10%(v/v) Al(OH)₃ (ALHYDROGEL, Superfos Biosector A/S, Denmark) on plasminogen recovery and PrP^{Sc} clearance during the PEG precipitation/depth filtration was determined. The protocol was as described above in Example 4, except that, following the addition of 3% PEG, 10% Al(OH)₃ (v/v) was added. The paste retained by the CUNO SP-30 filter was resuspended to original volume in TBS. Samples from the Prove (spiked extract prior to filtration), filtrate, and paste resuspension were analyzed for both plasminogen and PrP^{Sc} by Western analysis.

Adding Al(OH)₃ at the concentration indicated above resulted in an increase in PrP^{Sc} clearance of 2 logs (approximately 3 logs with versus 1 log without).

### Example 6 PrP^{Sc} Clearance Using Al(OH)₃ in Plasminogen Purification Process

The effect of 3% Al(OH)₃ on PrP^{Sc} clearance during processing of Caprylate Cake I (CCI) was determined.

CCI was extracted and processed as described above. In one experiment, both SBH spike and 3% Al(OH)₃ (v/v) were added prior to the cloth (porous polypropylene) filtration. Samples were removed from the Input (Prove) and cloth filtrate. The presence of PrP^{Sc} was determined in each sample by Western analysis.

Inclusion of 3% Al(OH)₃ (v/v) resulted in 2 logs of clearance of PrP^{Sc}.
Without Al(OH)₃, clearance was 0 logs.

### Example 7 Comparison of Fumed Silica and Fumed Alumina

In contrast to the Al(OH)₃ slurry utilized in the foregoing experiment, fumed alumina (SPECTRAL, Cabot Corporation, Tuscola, IL) was also used in experiments comparing its effect to fumed silica (CAB-O-SIL).

Fifty milliliters of 0.1% bovine serum albumin (BSA) in TBS was spiked with 0.5 ml of either clarified hamster scrapie brain homogenate or a highly purified (Bolton) spike. Following the spike, an initial 10 ml sample (Prove) was pulled for analysis to determine the initial titer of the material. Next, approximately 0.05 g of either fumed alumina or fumed silica was added to 15 ml conical tubes. To each of these tubes, 10 ml of the spiked material was added, and the tubes were inverted 15-20 times.

Filtration units were prepared by removing the plungers from three 10 ml syringes and fitting them with a 0.8 µm syringe filters. Three filtrations were performed for each of the spiking types. The first filtration had no metal oxide present, the second filtration had fumed alumina, and the third had fumed silica. The proves and resulting filtrates were analyzed by Western blot and found the following signals.

**Table 2 Comparison of Fumed Alumina to Fumed Silica for PrP Clearance**

| **Sample** | **Clarified PrP Spike*** | **Bolton Spike*** |
|---|---|---|
| ₁ Prove | 4.0 | 5.5 |
| 0.8 µm filter only | 3.5 | 3.0 |
| 0.5% fumed alumina | 0 | 0 |
| 0.5% fumed silica | 0 | 0 |

| | | |
|---|---|---|
| * Data represents log₁₀ values of PrP signal detection. | | |

Clearance to the limit of detection was observed for both metal oxides under both spiking conditions.

## Claims

1. A method of preparing a solution containing biological material, comprising
a) adding a metal oxide to biological material to obtain a solution comprising a mixture of the metal oxide and the biological material; and
b) separating the metal oxide from the mixture to form a resulting solution,
wherein pathogenic prion proteins possibly contaminating the biological material are substantially reduced in the resulting solution.

2. A method of preparing a solution containing biological material, comprising
a) adding a metal oxide to biological material to obtain a solution comprising a mixture of the metal oxide and the biological material;
b) separating the metal oxide from the mixture to form a resulting solution; and
c) evaluating the resulting solution for the presence or amount of pathogenic prion protein,
wherein pathogenic prion proteins possibly contaminating the biological material are substantially reduced in the resulting solution.

3. The method of claim 2, wherein the biological material is selected from the group consisting of blood-derived products, tissue-derived products, and recombinantly produced products.

4. The method of claim 2, wherein the biological material is a blood-derived product.

5. The method of claim 4, wherein the blood-derived product is of human origin.

6. The method of claim 4, wherein the blood-derived product is selected from the group consisting of immunoglobulins, blood coagulation factors, plasmin, plasminogen, α-1 proteinase inhibitor, and albumin.

7. The method of claim 2, wherein the metal oxide is selected from the group consisting of fumed silica and fumed alumina.

8. The method of claim 2, wherein the metal oxide is fumed silica.

9. The method of claim 8, wherein the fumed silica is **characterized by** a specific surface area of from about 130 m²/g to about 380 m²/g.

10. The method of claim 8, wherein the fumed silica is **characterized by** a specific surface area of from about 150 m²/g to about 300 m²/g.

11. The method of claim 8, wherein the fumed silica is **characterized by** a specific surface area of about 200 m²/g.

12. The method of claim 2, wherein separating the metal oxide from the mixture comprises filtration.

13. The method of claim 12, wherein the filtration comprises passing the mixture through a filtration system which retains particles larger than from about 0.1 µm to about 5 µm.

14. The method of claim 12, wherein the filtration comprises passing the mixture through a filtration system which retains particles larger than about 0.8 µm.

15. The method of claim 2, wherein evaluating the resulting solution for the presence or amount of pathogenic prion protein comprises evaluating a sample for infectivity using an assay selected from the group consisting of an animal bioassay or an immunoassay for the pathogenic prion protein.

16. The method of claim 2, wherein evaluating the resulting solution for the presence or amount of pathogenic prion protein comprises evaluating a sample for the presence of pathogenic prion protein using an immunoassay.

17. The method of claim 16, wherein the immunoassay is selected from the group consisting of Western blots and ELISA assays.

18. The method of claim 16, wherein the immunoassay is a Western blot.

19. The method of claim 2, wherein the metal oxide is aluminum hydroxide.

20. The method of claim 19, wherein the aluminum hydroxide, as a gel comprising about 2% by weight Al₂O_{3,} is present at a concentration from about 1% to about 10% by volume.

21. The method of claim 19, wherein the aluminum hydroxide, as a gel comprising about 2% by weight Al₂O₃, is present at a concentration from about 1% to about 5% by volume.

22. The method of claim 19, wherein the aluminum hydroxide, as a gel comprising about 2% by weight Al₂O₃, is present at a concentration of about 3% by volume.

23. A method of preparing a solution containing biological material, comprising
a) adding fumed silica **characterized by** a specific surface area of from about 150 m²/g to about 300 m²/g to biological material to obtain a solution comprising a mixture of fumed silica and the biological material;
b) separating the fumed silica from the mixture to form a resulting solution by passing the mixture through a filtration system comprising a filter which retains at least a substantial portion of particles of the fumed silica; and
c) evaluating the resulting solution for the presence or amount of pathogenic prion protein using an immunoassay,
wherein pathogenic prion proteins possibly contaminating the biological material are substantially reduced in the resulting solution.

24. The method of claim 23, wherein the fumed silica is **characterized by** a specific surface area of about 200 m²/g, a tap density of about 50 g/l and an average aggregate particle length of from about 0.2 µm to about 0.3 µm.

25. The method of claim 23, wherein the fumed silica is added in an amount from about 0.1% to about 1.0% (weight/weight) of the solution comprising a mixture of fumed silica and the biological material.

26. The method of claim 23, wherein the fumed silica is added in an amount from about 0.2% to about 0.8% (weight/weight) of the solution comprising a mixture of fumed silica and the biological material.

27. The method of claim 23, wherein the fumed silica is added in an amount of at least about 0.25% (weight/weight) of the solution comprising a mixture of fumed silica and the biological material.

28. The method of claim 23, wherein the fumed silica is added in an amount of at least about 0.5% (weight/weight) of the solution comprising a mixture of fumed silica and the biological material.

29. A method of preparing a solution containing biological material, comprising
a) adding silicon dioxide particles to biological material to obtain a solution comprising a mixture of silicon dioxide particles and the biological material;
b) separating the silicon dioxide particles from the mixture to form a resulting solution; and
c) evaluating the resulting solution for the presence or amount of pathogenic prion protein,
wherein pathogenic prion proteins possibly contaminating the biological material are substantially reduced in the resulting solution.

30. The method of claim 29, wherein separating the silicon dioxide particles from the mixture comprises centrifugation or filtration.

31. The method of claim 29, wherein separating the silicon dioxide particles from the mixture comprises centrifugation.

32. A method of separating prions from a sample, comprising
a) contacting a sample in a flowable liquid state with a solid substrate comprising a metal oxide;
b) allowing the sample to remain in contact with the substrate for a time such that prions in the sample bind to the substrate; and
c) separating the sample from the substrate.

33. The method of claim 32, wherein the metal oxide is silicon dioxide or aluminum hydroxide.

34. The method of claim 32, wherein the metal oxide is fumed silica.

35. A method of separating prion proteins from a sample and concentrating them for further analysis, the method comprising,
a) contacting the sample with a particulate metal oxide;
b) separating the particulate metal oxide from the sample;
c) subjecting prion proteins associated with the particulate metal oxide to further analysis.

36. The method of claim 35, wherein the further analysis of prion proteins comprises at least one analytical technique selected from the group consisting of immunoassay, animal bioassay, spectroscopic analysis, and chromatographic analysis.

## Patentansprüche

1. Verfahren zur Herstellung einer biologisches Material enthaltenden Lösung, umfassend:
a) das Zusetzen eines Metalloxids zu biologischem Material, um eine Lösung zu erhalten, die ein Gemisch aus dem Metalloxid und dem biologischen Material umfasst; und
b) das Trennen des Metalloxids vom Gemisch, um eine resultierende Lösung zu erhalten, worin pathogene Prionproteine, die möglicherweise das biologische Material verunreinigen, in der resultierenden Lösung deutlich verringert sind.

2. Verfahren zur Herstellung einer biologisches Material enthaltenden Lösung, umfassend:
a) das Zusetzen eines Metalloxids zu biologischem Material, um eine Lösung zu erhalten, die ein Gemisch aus dem Metalloxid und dem biologischen Material umfasst;
b) das Trennen des Metalloxids vom Gemisch, um eine resultierende Lösung zu erhalten; und
c) das Beurteilen der resultierenden Lösung bezüglich der Gegenwart oder Menge von pathogenem Prionprotein,
worin pathogene Prionproteine, die möglicherweise das biologische Material verunreinigen, in der resultierenden Lösung deutlich verringert sind.

3. Verfahren nach Anspruch 2, worin das biologische Material aus der Gruppe ausgewählt ist, die aus aus Blut stammenden Produkten, aus Gewebe stammenden Produkten und rekombinant hergestellten Produkte besteht.

4. Verfahren nach Anspruch 2, worin das biologische Material ein aus Blut stammendes Produkt ist.

5. Verfahren nach Anspruch 4, worin das aus Blut stammende Produkt menschlichen Ursprungs ist.

6. Verfahren nach Anspruch 4, worin das aus Blut stammende Produkt aus der aus Immunglobulinen, Blutgerinnungsfaktoren, Plasmin, Plasminogen, α-1-Proteinaseinhibitoren und Albumin bestehenden Gruppe ausgewählt ist.

7. Verfahren nach Anspruch 2, worin das Metalloxid aus der aus pyrogener Kieselsäure und pyrogenem Aluminiumoxid bestehenden Gruppe ausgewählt ist.

8. Verfahren nach Anspruch 2, worin das Metalloxid pyrogene Kieselsäure ist.

9. Verfahren nach Anspruch 8, worin die pyrogene Kieselsäure durch eine spezifische Oberfläche von etwa 130 m²/g bis etwa 380 m²/g **gekennzeichnet** ist.

10. Verfahren nach Anspruch 8, worin die pyrogene Kieselsäure durch eine spezifische Oberfläche von etwa 150 m²/g bis etwa 300 m²/g **gekennzeichnet** ist.

11. Verfahren nach Anspruch 8, worin die pyrogene Kieselsäure durch eine spezifische Oberfläche von etwa 200 m²/g **gekennzeichnet** ist.

12. Verfahren nach Anspruch 2, worin das Trennen des Metalloxids vom Gemisch Filtration umfasst.

13. Verfahren nach Anspruch 12, worin die Filtration das Durchführen des Gemischs durch ein Filtrationssystem umfasst, das Teilchen zurückhält, die größer als etwa 0,1 µm bis etwa 5 µm sind.

14. Verfahren nach Anspruch 12, worin die Filtration das Durchführen des Gemischs durch ein Filtrationssystem umfasst, das Teilchen zurückhält, die größer als etwa 0,8 µm sind.

15. Verfahren nach Anspruch 2, worin die Beurteilung der resultierenden Lösung auf die Gegenwart oder Menge von pathogenem Prionprotein das Beurteilen einer Probe auf Infektiosität unter Einsatz eines Tests umfasst, der aus der Gruppe ausgewählt ist, die einen Tier-Bioassay und einen Immunoassay für das pathogene Prionprotein umfasst.

16. Verfahren nach Anspruch 2, worin die Beurteilung der resultierenden Lösung auf die Gegenwart oder Menge von pathogenem Prionprotein das Beurteilen einer Probe auf die Gegenwart von pathogenem Prionprotein unter Einsatz eines Immunoassays umfasst.

17. Verfahren nach Anspruch 16, worin der Immunoassay aus der aus Western-Blots und ELISA-Assays bestehenden Gruppe ausgewählt ist.

18. Verfahren nach Anspruch 16, worin der Immunoassay ein Western-Blot ist.

19. Verfahren nach Anspruch 2, worin das Metalloxid Aluminiumhydroxid ist.

20. Verfahren nach Anspruch 19, worin das Aluminiumhydroxid, als etwa 2 Gew.-% Al₂O₃ umfassendes Gel, in einer Konzentration von etwa 1 bis etwa 10 Vol.-% vorhanden ist.

21. Verfahren nach Anspruch 19, worin das Aluminiumhydroxid, als etwa 2 Gew.-% Al₂O₃ umfassendes Gel, in einer Konzentration von etwa 1 bis etwa 5 Vol.-% vorhanden ist.

22. Verfahren nach Anspruch 19, worin das Aluminiumhydroxid, als etwa 2 Gew.-% Al₂O₃ umfassendes Gel, in einer Konzentration von etwa 3 Vol.-% vorhanden ist.

23. Verfahren zur Herstellung einer biologisches Material enthaltenden Lösung, umfassend:
a) das Zusetzen von pyrogener Kieselsäure, die durch eine spezifische Oberfläche von etwa 150 m²/g bis etwa 300 m²/g **gekennzeichnet** ist, zu biologischem Material, um eine Lösung zu erhalten, die ein Gemisch aus pyrogener Kieselsäure und dem biologischen Material umfasst;
b) das Trennen der pyrogenen Kieselsäure vom Gemisch, um eine resultierende Lösung zu erhalten, indem das Gemisch durch ein Filtrationssystem geführt wird, das ein Filter umfasst, welches zumindest einen wesentlichen Anteil der pyrogenen Kieselsäureteilchen zurückhält; und
c) das Beurteilen der resultierenden Lösung auf die Gegenwart oder Menge von pathogenem Prionprotein unter Einsatz eines Immunoassays,
worin pathogene Prionproteine, die möglicherweise das biologische Material verunreinigen, in der resultierenden Lösung deutlich verringert sind.

24. Verfahren nach Anspruch 23, worin die pyrogene Kieselsäure durch eine spezifische Oberfläche von etwa 200 m²/g, eine Klopfdichte von etwa 50g/l und eine mittlere Aggregatteilchenlänge von etwa 0,2 µm bis etwa 0,3 µm **gekennzeichnet** ist.

25. Verfahren nach Anspruch 23, worin die pyrogene Kieselsäure in einer Menge von etwa 0,1 bis etwa 1,0 Gew.-% der Lösung, die ein Gemisch aus pyrogener Kieselsäure und dem biologischen Material umfasst, zugesetzt wird.

26. Verfahren nach Anspruch 23, worin die pyrogene Kieselsäure in einer Menge von etwa 0,2 bis etwa 0,8 Gew.-% der Lösung, die ein Gemisch aus pyrogener Kieselsäure und dem biologischen Material umfasst, zugesetzt wird.

27. Verfahren nach Anspruch 23, worin die pyrogene Kieselsäure in einer Menge von zumindest etwa 0,25 Gew.-% der Lösung, die ein Gemisch aus pyrogener Kieselsäure und dem biologischen Material umfasst, zugesetzt wird.

28. Verfahren nach Anspruch 23, worin die pyrogene Kieselsäure in einer Menge von zumindest etwa 0,5 Gew.-% der Lösung, die ein Gemisch aus pyrogener Kieselsäure und dem biologischen Material umfasst, zugesetzt wird.

29. Verfahren zur Herstellung einer biologisches Material enthaltenden Lösung, umfassend:
a) das Zusetzen von Siliciumdioxidteilchen zu biologischem Material, um eine Lösung zu erhalten, die ein Gemisch aus Siliciumdioxidteilchen und dem biologischen Material umfasst;
b) das Trennen der Siliciumdioxidteilchen vom Gemisch, um eine resultierende Lösung zu erhalten; und
c) das Beurteilen der resultierenden Lösung auf die Gegenwart oder Menge von pathogenem Prionprotein,
worin pathogene Prionproteine, die möglicherweise das biologische Material verunreinigen, in der resultierenden Lösung deutlich verringert sind.

30. Verfahren nach Anspruch 29, worin das Trennen der Siliciumdioxidteilchen vom Gemisch Zentrifugation oder Filtration umfasst.

31. Verfahren nach Anspruch 29, worin das Trennen der Siliciumdioxidteilchen vom Gemisch Zentrifugation umfasst.

32. Verfahren zum Trennen von Prionen von einer Probe, umfassend:
a) das Kontaktieren einer Probe in einem fließfähigen flüssigen Zustand mit einem festen Substrat, das ein Metalloxid umfasst;
b) das In-Kontakt-Belassen der Probe mit dem Substrat für eine Dauer, die es den Prionen in der Probe erlaubt, an das Substrat zu binden; und
c) das Trennen der Probe vom Substrat.

33. Verfahren nach Anspruch 32, worin das Metalloxid Siliciumdioxid oder Aluminiumhydroxid ist.

34. Verfahren nach Anspruch 32, worin das Metalloxid pyrogene Kieselsäure ist.

35. Verfahren zum Trennen von Prionproteinen von einer Probe und ihr Einengen für eine weitere Analyse, wobei das Verfahren Folgendes umfasst:
a) das Kontaktieren der Probe mit einem partikulären Metalloxid;
b) das Trennen des partikulären Metalloxids von der Probe;
c) das Unterziehen der Prionproteine, die mit dem partikulären Metalloxid assoziiert sind, einer weiteren Analyse.

36. Verfahren nach Anspruch 35, worin die weitere Analyse von Prionproteinen zumindest ein Analyseverfahren umfasst, das aus der aus einem Immunoassay, einem Tier-Bioassay, einer spektroskopischen Analyse und einer chromatographischen Analyse bestehenden Gruppe ausgewählt ist.

## Revendications

1. Procédé de préparation d'une solution contenant une matière biologique, comprenant
a) ajouter un oxyde métallique à une matière biologique pour obtenir une solution comprenant un mélange de l'oxyde métallique et de la matière biologique ; et
b) séparer l'oxyde métallique du mélange pour former une solution résultante, où les protéines prions pathogènes contaminant éventuellement la matière biologique sont sensiblement réduites dans la solution résultante.

2. Procédé de préparation d'une solution contenant une matière biologique, comprenant
a) ajouter un oxyde métallique à une matière biologique pour obtenir une solution comprenant un mélange de l'oxyde métallique et de la matière biologique ;
b) séparer l'oxyde métallique du mélange pour former une solution résultante ; et
c) évaluer la solution résultante concernant la présence ou la quantité de protéines prions pathogènes,
où les protéines prions pathogènes contaminant éventuellement la matière biologique sont sensiblement réduites dans la solution résultante.

3. Procédé selon la revendication 2 où la matière biologique est choisie dans le groupe consistant en les produits dérivés du sang, les produits dérivés de tissus et les produits produits de manière recombinante.

4. Procédé selon la revendication 2 où la matière biologique est un produit dérivé du sang.

5. Procédé selon la revendication 4 où le produit dérivé du sang est d'origine humaine.

6. Procédé selon la revendication 4 où le produit dérivé du sang est choisi dans le groupe consistant en les immunoglobulines, les facteurs de coagulation sanguine, la plasmine, le plasminogène, l'inhibiteur d'α-1 protéinase et l'albumine.

7. Procédé selon la revendication 2 où l'oxyde métallique est choisi dans le groupe consistant en la silice fumée et l'alumine fumée.

8. Procédé selon la revendication 2 où l'oxyde métallique est la silice fumée.

9. Procédé selon la revendication 8 où la silice fumée est **caractérisée par** une surface spécifique d'environ 130 m²/g à environ 380 m²/g.

10. Procédé selon la revendication 8 où la silice fumée est **caractérisée par** une surface spécifique d'environ 150 m²/g à environ 300 m²/g.

11. Procédé selon la revendication 8 où la silice fumée est **caractérisée par** une surface spécifique d'environ 200 m²/g.

12. Procédé selon la revendication 2 où la séparation de l'oxyde métallique du mélange comprend une filtration.

13. Procédé selon la revendication 12 où la filtration comprend le passage du mélange au travers d'un système de filtration qui retient les particules de plus d'environ 0,1 µm à environ 5 µm.

14. Procédé selon la revendication 12 où la filtration comprend le passage du mélange au travers d'un système de filtration qui retient les particules de plus d'environ 0,8 µm.

15. Procédé selon la revendication 2 où l'évaluation de la solution résultante concernant la présence ou la quantité de protéines prions pathogènes comprend l'évaluation d'un échantillon concernant l'infectivité au moyen d'un essai choisi dans le groupe consistant en un bioessai sur des animaux ou un immunoessai pour les protéines prions pathogènes.

16. Procédé selon la revendication 2 où l'évaluation de la solution résultante concernant la présence ou la quantité de protéines prions pathogènes comprend l'évaluation d'un échantillon concernant la présence de protéines prions pathogènes au moyen d'un immunoessai.

17. Procédé selon la revendication 16 où l'immunoessai est choisi dans le groupe consistant en les transferts de Western et les essais ELISA.

18. Procédé selon la revendication 16 où l'immunoessai est un transfert de Western.

19. Procédé selon la revendication 2 où l'oxyde métallique est l'hydroxyde d'aluminium.

20. Procédé selon la revendication 19 où l'hydroxyde d'aluminium, sous forme d'un gel comprenant environ 2 % en masse de Al₂O₃, est présent à une concentration d'environ 1 % à environ 10 % en volume.

21. Procédé selon la revendication 19 où l'hydroxyde d'aluminium, sous forme d'un gel comprenant environ 2 % en masse de Al₂O₃, est présent à une concentration d'environ 1 % à environ 5 % en volume.

22. Procédé selon la revendication 19 où l'hydroxyde d'aluminium, sous forme d'un gel comprenant environ 2 % en masse de Al₂O₃, est présent à une concentration d'environ 3 % en volume.

23. Procédé de préparation d'une solution contenant une matière biologique, comprenant
a) ajouter de la silice fumée **caractérisée par** une surface spécifique d'environ 150 m²/g à environ 300 m²/g à une matière biologique pour obtenir une solution comprenant un mélange de silice fumée et de la matière biologique ;
b) séparer la silice fumée du mélange pour former une solution résultante par passage du mélange au travers d'un système de filtration comprenant un filtre qui retient au moins une partie sensible des particules de la silice fumée ; et
c) évaluer la solution résultante concernant la présence ou la quantité de protéines prions pathogènes au moyen d'un immunoessai,
où les protéines prions pathogènes contaminant éventuellement la matière biologique sont sensiblement réduites dans la solution résultante.

24. Procédé selon la revendication 23 où la silice fumée est **caractérisée par** une surface spécifique d'environ 200 m²/g, une masse volumique tassée d'environ 50 g/l et une longueur moyenne de particule d'agrégat d'environ 0,2 µm à environ 0,3 µm.

25. Procédé selon la revendication 23 où la silice fumée est ajoutée en une quantité d'environ 0,1 % à environ 1,0 % (masse/masse) de la solution comprenant un mélange de silice fumée et de la matière biologique.

26. Procédé selon la revendication 23 où la silice fumée est ajoutée en une quantité d'environ 0,2 % à environ 0,8 % (masse/masse) de la solution comprenant un mélange de silice fumée et de la matière biologique.

27. Procédé selon la revendication 23 où la silice fumée est ajoutée en une quantité d'au moins environ 0,25 % (masse/masse) de la solution comprenant un mélange de silice fumée et de la matière biologique.

28. Procédé selon la revendication 23 où la silice fumée est ajoutée en une quantité d'au moins environ 0,5 % (masse/masse) de la solution comprenant un mélange de silice fumée et de la matière biologique.

29. Procédé de préparation d'une solution contenant une matière biologique, comprenant
a) ajouter des particules de dioxyde de silicium à une matière biologique pour obtenir une solution comprenant un mélange de particules de dioxyde de silicium et de la matière biologique ;
b) séparer les particules de dioxyde de silicium du mélange pour former une solution résultante; et
c) évaluer la solution résultante concernant la présence ou la quantité de protéines prions pathogènes,
où les protéines prions pathogènes contaminant éventuellement la matière biologique sont sensiblement réduites dans la solution résultante.

30. Procédé selon la revendication 29 où la séparation des particules de dioxyde de silicium du mélange comprend une centrifugation ou une filtration.

31. Procédé selon la revendication 29 où la séparation des particules de dioxyde de silicium du mélange comprend une centrifugation.

32. Procédé de séparation de prions d'un échantillon, comprenant
a) mettre en contact un échantillon dans un état liquide fluide avec un substrat solide comprenant un oxyde métallique ;
b) permettre à l'échantillon de rester en contact avec le substrat pendant une durée telle que les prions dans l'échantillon se lient au substrat ; et
c) séparer l'échantillon du substrat.

33. Procédé selon la revendication 32 où l'oxyde métallique est le dioxyde de silicium ou l'hydroxyde d'aluminium.

34. Procédé selon la revendication 32 où l'oxyde métallique est la silice fumée.

35. Procédé pour séparer des protéines prions d'un échantillon et pour les concentrer en vue d'une analyse supplémentaire, le procédé comprenant
a) mettre en contact l'échantillon avec un oxyde métallique particulaire ;
b) séparer l'oxyde métallique particulaire de l'échantillon ;
c) soumettre les protéines prions associées avec l'oxyde métallique particulaire à une analyse supplémentaire.

36. Procédé selon la revendication 35 où l'analyse supplémentaire des protéines prions comprend au moins une technique analytique choisie dans le groupe consistant en un immunoessai, un bioessai sur des animaux, l'analyse spectroscopique et l'analyse chromatographique.
